(19) Europäisches Patentamt
European Patent Office
Office européen des brevets

(11) **EP 4 056 204 B1**

(12) **EUROPEAN PATENT SPECIFICATION**

(45) Date of publication and mention
of the grant of the patent:
**09.10.2024 Bulletin 2024/41**

(21) Application number: **21899301.2**

(22) Date of filing: **05.02.2021**

(51) International Patent Classification (IPC):
**A61L 24/08** (2006.01)  **A61L 24/00** (2006.01)
**C08J 3/09** (2006.01)  **A61L 26/00** (2006.01)

(52) Cooperative Patent Classification (CPC):
(C-Sets available)
**A61L 24/0036; A61L 24/08; A61L 26/0023;
A61L 26/0085; C08J 3/091;** A61L 2400/04;
C08J 2303/04; C08J 2371/02; C08J 2403/02

(Cont.)

(86) International application number:
**PCT/CN2021/075416**

(87) International publication number:
**WO 2022/147877 (14.07.2022 Gazette 2022/28)**

(54) **STARCH-BASED FLUFFY PARTICLES AND PREPARATION METHOD THEREFOR AND APPLICATION THEREOF**

FLOCKIGE TEILCHEN AUF STÄRKEBASIS, VERFAHREN ZU IHRER HERSTELLUNG UND IHRE ANWENDUNG

PARTICULES DUVETEUSES À BASE D'AMIDON, ET PROCÉDÉ DE PRÉPARATION ET APPLICATION ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.01.2021  CN 202110008377**

(43) Date of publication of application:
**14.09.2022 Bulletin 2022/37**

(73) Proprietor: **HONEST MEDICAL CHINA CO., LTD.**
**Zhuhai, Guangdong 519031 (CN)**

(72) Inventors:
• **TANG, Shunqing**
**Zhuhai, Guangdong 519031 (CN)**
• **WANG, Jianjin**
**Zhuhai, Guangdong 519031 (CN)**
• **GUO, Aijun**
**Zhuhai, Guangdong 519031 (CN)**

(74) Representative: **Primiceri, Maria Vittoria et al**
**Praxi Intellectual Property S.p.A.**
**Via Leonida Bissolati, 20**
**00187 Roma (IT)**

(56) References cited:
EP-A1- 1 178 843     CN-A- 101 423 620
CN-A- 101 423 620    CN-A- 101 559 235
CN-A- 105 617 449    CN-A- 105 688 265
CN-A- 106 377 792    JP-A- S 623 752
KR-B1- 101 849 928   US-A1- 2020 405 907

• **DATABASE WPI Week 198707, 9 January 1987 Derwent World Patents Index; AN 1987-046422, XP002809349**
• **DATABASE WPI Week 201751, 18 April 2018 Derwent World Patents Index; AN 2017-39858K, XP002809350**

- SOLEIMANPOUR MARJAN ET AL: "Fabrication of nanostructured mesoporous starch encapsulating soy-derived phytoestrogen (genistein) by well-tuned solvent exchange method", INTERNATIONAL JOURNAL OF BIOLOGICAL MACROMOLECULES, ELSEVIER BV, NL, vol. 159, 19 May 2020 (2020-05-19), pages 1031 - 1047, XP086248333, ISSN: 0141-8130, [retrieved on 20200519], DOI: 10.1016/J.IJBIOMAC.2020.05.124
- JAMES PATINDOL ET AL: "Porous rice powder from the precipitation of gelatinized flour or starch paste with ethanol", STARCH/STARKE, WILEY-VCH VERLAG, WEINHEIM, DE, vol. 65, no. 3-4, 1 March 2013 (2013-03-01), pages 1 - 8, XP002729758, ISSN: 0038-9056, [retrieved on 20121119], DOI: 10.1002/STAR.201200134
- SADEGHI ROHOLLAH ET AL: "Effects of starch composition and type of non-solvent on the formation of starch nanoparticles and improvement of curcumin stability in aqueous media", JOURNAL OF CEREAL SCIENCE, ACADEMIC PRESS LTD, GB, vol. 76, 9 June 2017 (2017-06-09), pages 122 - 130, XP085155807, ISSN: 0733-5210, DOI: 10.1016/J.JCS.2017.05.020
- SAARI HISFAZILAH ET AL: "Production of starch nanoparticles by dissolution and non-solvent precipitation for use in food-grade Pickering emulsions", CARBOHYDRATE POLYMERS, APPLIED SCIENCE PUBLISHERS , LTD BARKING, GB, vol. 157, 4 October 2016 (2016-10-04), pages 558 - 566, XP029848615, ISSN: 0144-8617, DOI: 10.1016/J.CARBPOL.2016.10.003
- DATABASE WPI Week 200937, 6 May 2009 Derwent World Patents Index; AN 2009-J13505, XP002809351
- HAN LI;ZHANG DINGKUN;ZHANG FANG;YANG YINGGUANG;YANG MING: "Preparation of Porous Starch and Research Progress in Medical Application", CHINESE TRADITIONAL PATENT MEDICINE, vol. 37, no. 6, 20 June 2015 (2015-06-20), pages 1321 - 1324, XP055949815, ISSN: 1001-1528, DOI: 10.3969/j.issn.1001-1528.2015.06.036
- BIAN WENHAI;LIU CHENGCHENG;LI QIAN;WANG CHAOZHAN;BIAN LIUJIAO: "Research on the Hemostatic Preparation with Corn Microporous Starch", CURRENT BIOTECHNOLOGY, vol. 7, no. 4, 25 July 2017 (2017-07-25), pages 320 - 326+354-355, XP055949819, ISSN: 2095-2341, DOI: 10.19586/j.2095-2341.2016.0126
- TIAN MENGYI;ZHENG YUZHEN;REN HUIMING;LI SAI: "Development of Starch-Based Hemostatic Materials", BEIJING BIOMEDICAL ENGINEERING, vol. 38, no. 4, 15 August 2019 (2019-08-15), pages 434 - 438, XP055949824, ISSN: 1002-3208, DOI: 10.3969/j.issn.1002-3208.2019.04.015

(52) Cooperative Patent Classification (CPC): (Cont.)

C-Sets
A61L 24/08, C08L 3/02;
A61L 26/0023, C08L 3/02

**Description**

**TECHNICAL FIELD**

The present disclosure relates to the technical field of hemostatic materials, and more particularly, relates to a starch-based fluffy particle, a preparation method and use thereof.

**BACKGROUND**

A hemostatic procedure in surgical trauma and operation is an important factor in clinic, in which a hemostatic material plays an important role. At present, a starch-based material is widely used as the hemostatic material due to a good biological safety, a high water absorption rate, a degradability, and the like. An original particle size of starch particles derived from plants such as corns and potatoes generally ranges from 10 $\mu$m to 50 $\mu$m, and the particle size of most of the particles is less than 30 $\mu$m. The particle size of the starch particles does not change greatly even after chemical modification (etherification, gelatinization, crosslinking or carboxymethylation). When these small-sized starch particles are aggregated, a capillary phenomenon occurs due to a small gap after aggregation of the particles, which leads to excellent water absorption. Due to the existence of air between the particles, it will hinder the water absorption caused by the capillary phenomenon, which leads to poor overall water absorption and easy aggregation during water absorption, thus resulting in difficult subsequent water penetration, so that a whole starch particle aggregate has a slow water absorption speed and a greatly reduced water absorption rate. When used in special occasions, for example, when used as hemostatic powder for spraying hemostasis, the starch particles have a poor hemostatic effect due to slow water absorption speed and low total water absorption rate.

Existing researches show that the particle size of the starch particles and the chemical structure of starch may both affect the water absorption speed and the water absorption rate of the starch aggregate. Generally, carboxymethylated starch or etherified starch with the same particle size has a higher water absorption rate and a faster water absorption speed than original starch. Starch aggregate with different particle sizes or aggregation forms prepared from the starch with the same chemical structure also have difference in water absorption speed and water absorption rate. Arista™ modified starch hemostatic powder produced by Medafor Company of America has a particle diameter less than 50 $\mu$m, and although the hemostatic powder has a porous particle surface, the hemostatic powder has a low water absorption speed and a low water absorption rate. In Chinese Patent CN200710141944.0 to Xin Ji, et al., a porous spherical aggregate having a particle size ranged from 10 $\mu$m to 1,000 $\mu$m, and preferably ranged from 50 $\mu$m to 500 $\mu$m is prepared by using original particles of modified starch, and it is found that a water absorption speed and a water absorption rate were greatly improved in the case of large particles.

There are many related starch hemostatic particle products, for example, HaemoCer starch particle hemostatic powder (composed of carboxymethylated potato starch) from BIOCER Company of Germany, whose particle size is10 $\mu$m to 50 $\mu$m, although a water absorption speed is fast in an initial stage and a water absorption rate is high (20 times), a water retention capacity is insufficient in a later stage, and gel is easy to be thin to flow. Natural polysaccharide microspheres invented by Wenzhou Institute of Biomaterials and Engineering in China are prepared by emulsifying and dispersing, physically crosslinking into spheres, and vacuum drying at 60°C to obtain microspheres with an adjustable particle size of 20 $\mu$m to 50 $\mu$m for hemostasis, but the hemostatic effect of the microspheres is still insufficient. CN 101 423 620 A (2009-05-06) discloses a preparation method comprising the process steps of centrifugation and freeze drying but the resulting product is not satisfactory and a material with improve characteristics is still needed, particularly in terms of water absorption rate and speed as well as gel strength properties.

Although existing starch-based hemostatic materials have been improved by various methods, the prepared large-particle modified starch-based hemostatic materials still cannot fully meet actual clinical hemostatic requirements. Therefore, it is urgent to develop a starch-based hemostatic material that has a high hemostatic performance, and can implement hemostasis of traumatic wounds by coating or spraying, or is used for hemostasis of in-vivo micro-leakage under endoscope/minimal invasion.

**SUMMARY**

The present disclosure aims to solve at least one of the technical problems in the prior art above. Therefore, the present disclosure provides a starch-based fluffy particle, a preparation method and use thereof. The starch-based fluffy particles provided are porous and fluffy with a large particle gap, have excellent water absorption speed, water absorption rate and degradation rate, and can achieve the purpose of rapid and efficient hemostasis. In addition, the starch-based fluffy particles are free of dispersing agent, emulsifying agent or cross-linking agent, have the characteristics of safety and reliability, and can meet the hemostatic requirement of in-vivo environment.

A preparation method of a starch-based fluffy particle, comprises the following steps of:

(1) adding water into starch or modified starch for gelatinization to obtain a starch paste;

(2) adding the starch paste into an organic solvent that does not dissolve the starch paste so that the starch paste is dispersed into several precipitates in the organic solvent and the precipitates are swelled, stirring, standing and centrifuging to collect precipitates; and

(3) freeze-drying, crushing and sieving the precipitates in sequence to prepare the starch-based fluffy particle.

In the related prior art, the prepared starch paste is usually directly freeze-dried to prepare a hemostatic sponge-like or lumpy aggregate structure, and then crushed to finally prepare hemostatic particles. However, after adding water into starch or modified starch for gelatinization, starch particles swell, molecular chains on particle surfaces stretch after water absorption and contact with each other, and diffuse to entangle partially, and at the moment, the starch particles lean against each other to form a whole adhesive body. The strength of the formed starch paste before freeze-drying is not high, and a flexible lumpy or sponge-like product may be formed during freeze-drying, so starch molecules in the formed hemostatic sponge are entangled with each other and difficult to be dispersed, which is not beneficial for subsequent crushing into uniform small particles. Moreover, the water absorption speed and the water absorption rate of the prepared particles are poor, so that the hemostatic requirement of practical use cannot be met.

In addition, there are some starch hemostatic materials on the market, which are formed into spheres by adding the crosslinking agent and the emulsifying agent into the starch paste. The starch molecules can be linked in a form of chemical bond by introducing the cross-linking agent to form a compact and stable structure, which can reduce the swelling rate and the in-vivo degradation rate. However, since the cross-linking agent is insoluble in water, it is necessary to add the organic solvent as an assistant to dissolve the cross-linking agent to make it work. The organic solvent also plays a role of adjusting the polarity of the reaction solution, which is convenient for the cross-linking agent to fully react with main ingredients of hemostatic powder. The hemostatic particles prepared by this method have the advantages of a long in-vivo retention time, a slow degradation speed and a stable property, and the disadvantages of more compact original starch particles caused by cross-linking, an enhanced hydrophobicity of the starch, and small pores inside and between particles, leading to a greatly reduced water absorption rate and a slow water absorption speed. Meanwhile, the degradation time of the hemostatic particles is prolonged due to cross-linking, which leads to the prepared hemostatic particles not meeting regulations of finishing hemostasis and degradation within 48 hours in a digestive tract or minimally invasive operation, so that the use of the hemostatic particles is limited. Thus, it can be seen that when the cross-linking agent, the emulsifying agent and other ingredients are not used in a starch material, there is no motivation or reason for adding the organic solvent.

However, the present disclosure first discovers and points out that, by using the organic solvent to stir and disperse the starch paste before freeze-drying, the following effects can be achieved: 1. by introducing the organic solvent insoluble with the starch paste, and then stirring, the starch paste may be dispersed into several granular precipitates, which is convenient for drying and crushing, to obtain uniform particles. 2. The organic solvent has a dehydration effect, which can effectively reduce a water content between the precipitates during freezing to avoid adhesion phenomenon caused by the re-entanglement of the precipitates due to the presence of water during freezing and drying. The precipitates are stable and hard, which is convenient for subsequent crushing and ball milling. 3. Furthermore, the organic solvent may penetrate inside the precipitates to further swell the precipitates, so that pores are also formed inside the dried precipitates to form a porous fluffy structure, thus effectively improving the water absorption speed and the water absorption rate of the starch-based material.

In the preparation method, after finishing the stirring in step (2), by standing for a period of time, water molecules diffuses into the precipitates, and the organic solvent penetrates into the interior of the precipitates and swells the precipitates to disperse the starch molecules in the precipitates, thus further improving the fluffy degree.

Preferably, in step (1), the modified starch is at least one selected from the group consisting of etherified starch, carboxymethylated starch, esterified starch and crosslinked starch.

Preferably, in step (1), a mass ratio of the starch or the modified starch to the water in the starch paste is from 1:3 to 1:40.

Preferably, in step (2), the organic solvent is at least one selected from the group consisting of methanol, ethanol, propanol, butanol, acetone, isopropanol, and dimethyl sulfoxide. More preferably, the organic solvent is isopropanol, methanol, ethanol, a combination of isopropanol and methanol, dimethyl sulfoxide or acetone. Experiments show that when the organic solvent is isopropanol, methanol, ethanol, the combination of isopropanol and methanol, dimethyl sulfoxide or acetone, the prepared starch-based fluffy particle has a higher fluffy degree and a better water absorption effect.

Preferably, the starch-based fluffy particle further comprises a hydrophilic polymer material. Addition of the hydrophilic polymer material may affect the stability, adhesion and hemostasis properties of the product to a certain extent, and the hydrophilic polymer material is, but is not limited to sodium carboxymethyl cellulose, hyaluronic acid, polyoxyethylene or collagen. In some embodiments, the hydrophilic polymer material is at least one selected form group consisting of sodium carboxymethyl cellulose, hyaluronic acid, polyoxyethylene, and collagen.

Preferably, in step (2), the organic solvent further comprises water. Due to an actual preparation requirement or the difficulty in obtaining a pure organic solvent, based on the total amount of the organic solvent and the water being 100vol%, the amount of the water is about 0vol% to 50vol%.

Preferably, in step (2), the stirring is performed at a rotating speed of 100 r/min to 3,000 r/min, and lasts for 0.5 hour to 2 hours.

Preferably, in step (2), the centrifuging is performed at a rotating speed of 2,000 r/min to 5,000 r/min. The centrifuging is intended to remove a part of organic solvent, ora part of organic solvent and water.

Preferably, in step (3), the freeze-drying comprises the steps of: freezing the precipitates at -30°C to -10°C for 3 hours to 24 hours first, and then freeze-drying the precipitates in a freeze dryer at -50°C to -30°C for 15 hours to 36 hours. Solid ice (bound water) and residual organic solvent may be directly removed by using the vacuum freeze-drying process, and freezing of the water under the freezing condition may further swell the particles, thus improving the fluffy degree. In addition, the original fluffy state of the particles may be kept by low-temperature vacuum drying, which can better keep the porous structure inside and between the starch particles.

A starch-based fluffy particle prepared by the preparation method above is provided, wherein a particle size of the starch-based fluffy particle is from *30 μm to 200 μm.*

Use of the starch-based fluffy particle above as a medical hemostatic material is provided. The starch-based fluffy particle may be coated or sprayed on a traumatic wound as hemostatic powder for hemostasis, and may also be applied to a digestive tract wound and a minimally invasive operation for hemostasis.

Compared with the prior art, the present disclosure has the following beneficial effects:

(1) In the present disclosure, the organic solvent and the water are used to prepare the starch-based fluffy particle, and the organic solvent and the water have a strong volatility, thus being easy to remove during freeze-vacuum drying, and having little residue. In addition, in the present disclosure, the chemical dispersing agent, the emulsifying agent, the cross-linking agent and other ingredients are not added, thus the starch-based fluffy particle is safer and more reliable, and capable of meeting hemostatic requirements of the digestive tract wound and the minimally invasive operation.

(2) By using the organic solvent, the water in the collected precipitate is more easily removed, and the collected precipitate is more brittle after freeze-drying to remove the water, and is easy to be crushed into the starch-based fluffy particles of a required size. Compared with the starch aggregates with the same chemical structure, the finally obtained starch-based fluffy particle is also significantly improved in water absorption speed and water absorption rate.

(3) In the present disclosure, the modified starch may be used as the starch raw material to further improve the water absorption rate, the water absorption speed, the adhesion property, the gel strength and other properties of the starch-based fluffy particles.

## BRIEF DESCRIPTION OF THE DRAWINGS

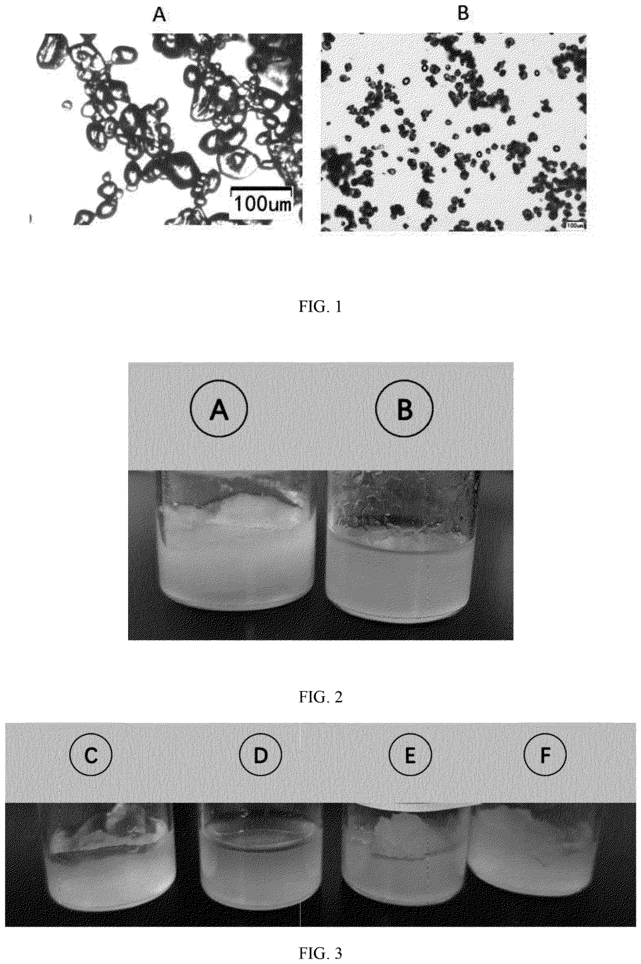

FIG. 1 shows the micro-structure of dried carboxymethyl corn starch-based precipitates in Example 1 and the micro-structure of dried carboxymethyl corn starch-based precipitates in Example 1 after crushing, wherein A represents the micro-structure of the dried carboxymethyl corn starch-based precipitates in Example 1, and B represents the micro-structure of the dried carboxymethyl corn starch-based precipitates in Example 1 after crushing.

FIG. 2 shows the results of the carboxymethyl corn starch-based fluffy particles in Example 1 and the carboxymethyl corn starch raw powder after 20 times of water adsorption, wherein A represents the carboxymethyl corn starch-based fluffy particles, and B represents the carboxymethyl corn starch raw powder.

FIG. 3 shows the gel stability results of different types of starch particles after 20 times of water adsorption, wherein C represents a German hemostatic powder (hemostatic powder prepared from potato starch by a cross-linking technology), D represents the carboxymethyl potato starch without any treatment, E represents the carboxymethyl potato starch-based fluffy particles prepared in Example 2, and F represents the carboxymethyl corn starch-based fluffy particles prepared in Example 1.

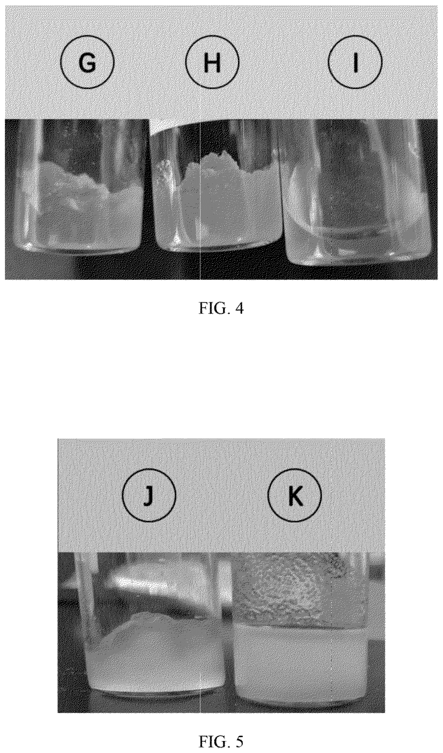

FIG. 4 shows the gel stability results of different types of starch particles after water adsorption and standing for 48 hours, wherein G represents the carboxymethyl potato starch-based fluffy particles prepared in Example 2, H represents the carboxymethyl corn starch-based fluffy particles prepared in Example 1, and I represents the German product (the hemostatic powder prepared from potato starch by a cross-linking technology).

FIG. 5 shows the gel stability results of the carboxymethyl potato starch-based fluffy particles prepared in Example 2 and a Chinese market product after 20 times of water adsorption, wherein J represents the carboxymethyl potato starch-based fluffy particles prepared in Example 2, and K represents the Chinese market product (a product prepared according to the Chinese patent CN200610053680.9).

## DETAILED DESCRIPTION

In order to make those skilled in the art better understand the technical solutions of the present disclosure, the following examples are now provided for description. It should be pointed out that the following examples do not limit the scope of protection claimed in the present disclosure.

Unless otherwise specified, the raw materials, reagents or devices used in the following examples may all be obtained in conventional commercial ways, or obtained by existing known methods.

Example 1

The example provided a carboxymethyl corn starch-based fluffy particle, and a preparation method of the carboxymethyl corn starch-based fluffy particle comprised the following steps.

(1) 1 g of carboxymethyl corn starch with a degree of substitution of 0.24 was added into 10 mL of distilled water, and stirred evenly at room temperature to prepare a carboxymethyl corn starch paste.

(2) An aqueous solution containing 95% methanol was prepared, and the carboxymethyl corn starch paste was transferred into 20 mL of methanol solution at a stirring speed of 1,000 r/min and room temperature, continuously stirred for 0.5 hour after finishing transferring, and then stood for 3 hours. The supernatant liquid was dumped, the lower layer containing the solvent and the starch-based precipitates was centrifuged at a rotating speed of 3,000 r/min to remove most of the solvent adsorbed on the starch-based precipitate, and the starch-based precipitates were collected.

(3) The collected starch-based precipitates were frozen in a refrigerator at -20°C for 15 hours, and then freeze-dried in a freeze dryer at -50°C for 20 hours to obtain dried carboxymethyl corn starch-based precipitates.

(4) The dried carboxymethyl corn starch-based precipitates were crushed, and then sieved with an 80-mesh sieve to obtain crushed precipitates with a particle size less than 200 $\mu$m. Then, the obtained crushed precipitates were sieved with a 300-mesh sieve to remove the crushed precipitates with a particle size less than 50 $\mu$m, so as to obtain carboxymethyl corn starch-based fluffy particles with a particle size of 50 $\mu$m to 200 $\mu$m.

In FIG. 1, A represents the micro-structure of the dried carboxymethyl corn starch-based precipitates obtained in this example, and in FIG. 1, B represents the micro-structure of the dried carboxymethyl corn starch-based precipitates obtained in this example after crushing. It can be seen from FIG. 1 that the starch particles inside the dried carboxymethyl corn starch-based precipitates are entangled and connected with each other, with a porous interior, and there are large gaps between the dried carboxymethyl corn starch-based precipitates, so that it is not easy to cause a capillary phenomenon.

in FIG. 2, A represents the result of the carboxymethyl corn starch-based fluffy particles prepared this example after 20 times of water adsorption, and in FIG. 2, B represents the result of the carboxymethyl corn starch raw powder after 20 times of water adsorption. It can be seen from FIG. 2 that the carboxymethyl corn starch raw powder is in a flowing state, while the carboxymethyl corn starch-based fluffy particles prepared in this example can still be kept in a certain form, which indicates that an upper limit of water adsorption is still not reached. Another experiment shows that, compared with the carboxymethyl corn starch raw powder, the water absorption rate of the carboxymethyl corn starch-based fluffy particles prepared in this example is increased by more than 4 times on average, and the water absorption speed is accelerated, which can be reduced from 35 seconds to 10 seconds on average.

Example 2

The example provided a carboxymethyl potato starch-based fluffy particle, and a preparation method of the carboxymethyl potato starch-based fluffy particle comprised the following steps.

(1) 1 g of carboxymethyl potato starch with a degree of substitution of 0.3 was added into 10 mL of distilled water, and stirred evenly at room temperature to prepare a carboxymethyl potato starch paste.

(2) An aqueous solution containing 90% ethanol was prepared, and the carboxymethyl potato starch paste was transferred into 20 mL of ethanol solution at a stirring speed of 600 r/min and room temperature, continuously stirred for 0.5 hour after finishing transferring, and then stood for 3 hours. The supernatant liquid was dumped, the lower layer containing the solvent and the starch-based precipitates was centrifuged at a rotating speed of 2,000 r/min to remove most of the solvent adsorbed on the starch-based precipitates, and the starch-based precipitates were collected.

(3) The collected starch-based precipitates were frozen in a refrigerator at -28°C for 20 hours, and then freeze-dried in a freeze dryer at -50°C for 24 hours to obtain dried carboxymethyl potato starch-based precipitates.

(4) The dried carboxymethyl potato starch-based precipitates were crushed, and then sieved with an 80-mesh sieve to obtain crushed precipitates with a particle size less than 200 $\mu$m. Then, the obtained crushed precipitates were sieved with a 350-mesh sieve to remove the crushed precipitates with a particle size less than 30 $\mu$m, so as to obtain carboxymethyl potato starch-based fluffy particles with a particle size of 30 $\mu$m to 200 $\mu$m.

When the carboxymethyl potato starch-based fluffy particles prepared in this example were used in a hemostasis experiment of a broken tail of a mouse. The bleeding volume of the mouse during hemostasis was low, and the hemostasis time was short. Complete hemostasis could be realized within 100 seconds on average, and the hemostasis speed was much faster than that of Chinese and foreign hemostatic powder products that have a hemostasis time of more than 210 seconds.

Example 3

The example provided a polyoxyethylene/carboxymethyl potato starch-based fluffy particle, and a preparation method of the polyoxyethylene/carboxymethyl potato starch-based fluffy particle comprised the following steps.

(1) 0.1 g of polyoxyethylene powder with a molecular weight of 50,000 was dispersed in 20 mL of distilled water, and then 1 g of carboxymethyl potato starch with a degree of substitution of 0.3 was added, and stirred evenly at room temperature to prepare a polyoxyethylene/carboxymethyl potato starch paste.

(2) 50 mL of aqueous solution containing 95% isopropanol was prepared, and the polyoxyethylene/carboxymethyl potato starch paste was transferred into the 50 mL isopropanol solution at a stirring speed of 1,500 r/min and room temperature, continuously stirred for 0.5 hour after finishing transferring, and then stood for 3 hours. The supernatant liquid was dumped, the lower layer containing the solvent and the starch-based precipitates was centrifuged at a rotating speed of 3,000 r/min to remove most of the solvent adsorbed on the starch-based precipitates, and the starch-based precipitates were collected.

(3) The collected starch-based precipitates were frozen in a refrigerator at -30°C for 10 hours, and then freeze-dried in a freeze dryer at -50°C for 24 hours to obtain dried polyoxyethylene/carboxymethyl potato starch-based precipitates.

(4) The dried polyoxyethylene/carboxymethyl potato starch-based precipitates were crushed, and then sieved with an 80-mesh sieve to obtain crushed precipitates with a particle size less than 200 $\mu$m. Then, the obtained crushed precipitates were sieved with a 300-mesh sieve to remove the crushed precipitates with a particle size less than 50 $\mu$m, so as to obtain polyoxyethylene/carboxymethyl potato starch-based fluffy particles with a particle size of 50 $\mu$m to 200 $\mu$m.

In this example, the flexibility of carboxymethyl potato starch gel was further improved by adding polyoxyethylene into the carboxymethyl potato starch.

Example 4

The example provided a carboxymethyl potato starch-based fluffy particle, and a preparation method of the carboxymethyl potato starch-based fluffy particle comprised the following steps.

(1) 1 g of carboxymethyl potato starch with a degree of substitution of 0.3 was added into 10 mL of distilled water, and stirred evenly at room temperature to prepare a carboxymethyl potato starch paste.

(2) An aqueous solution containing 50% isopropanol and 45% methanol (i.e., an isopropanol-methanol aqueous solution) was prepared, and the carboxymethyl potato starch paste was transferred into 20 mL of isopropanol-methanol solution at a stirring speed of 600 r/min and room temperature, continuously stirred for 0.5 hour after finishing transferring, and then stood for 3 hours. The supernatant liquid was dumped, the lower layer containing the solvent and the starch-basedprecipitates was centrifuged at a rotating speed of 2,000 r/min to remove most of the solvent adsorbed on the starch-based precipitates, and the starch-based precipitates were collected.

(3) The collected starch-based precipitates were frozen in a refrigerator at -28°C for 20 hours, and then freeze-dried in a freeze dryer at -50°C for 24 hours to obtain dried carboxymethyl potato starch-based precipitates.

(4) The dried carboxymethyl potato starch-based precipitates were crushed, and then sieved with an 80-mesh sieve to obtain crushed precipitates with a particle size less than 200 $\mu$m. Then, the obtained crushed precipitates were sieved with a 350-mesh sieve to remove the crushed precipitates with a particle size less than 30 $\mu$m, so as to obtain carboxymethyl potato starch-based fluffy particles with a particle size of 30 $\mu$m to 200 $\mu$m.

Product effect test

FIG. 3 shows the gel stability results of different types of starch particles after 20 times of water adsorption, wherein C represents a German hemostatic powder (hemostatic powder prepared from potato starch by a cross-linking technology), D represents the carboxymethyl potato starch without any treatment, E represents the carboxymethyl potato starch-based fluffy particles prepared in Example 2, and F represents the carboxymethyl corn starch-based fluffy particles prepared in Example 1. It can be seen from FIG. 3 that the carboxymethyl potato starch without any treatment is in a flowing state, with a poor water absorption capacity, and the performance of the carboxymethyl potato starch without any treatment is far inferior to those of the German hemostatic powder and the starch-based fluffy particles prepared in Examples 1 to 2.

FIG. 4 shows the gel stability results of different types of starch particles after water adsorption and standing for 48 hours, wherein G represents the carboxymethyl potato starch-based fluffy particles prepared in Example 2, H represents the carboxymethyl corn starch-based fluffy particles prepared in Example 1, and I represents the German product (the hemostatic powder prepared from potato starch by the cross-linking technology). It can be seen from FIG. 4 that the German product is melt to flow, which indicates that the gel stability of the German product is not as good as those of the starch-based fluffy particles prepared in Examples 1 to 2.

FIG. 5 shows the gel stability results of the carboxymethyl potato starch-based fluffy particles prepared in Example 2 and a Chinese market product after 20 times of water adsorption, wherein J represents the carboxymethyl potato starch-based fluffy particles prepared in Example 2, and K represents the Chinese market product (a product prepared according to the Chinese patent CN200610053680.9). It can be seen from FIG. 5 that the carboxymethyl potato starch-based fluffy particles prepared in Example2 are gel-like after water absorption, while the Chinese market product is dilute suspension, which indicates that the starch-based fluffy particles of the present disclosure have a better gel stability.

Tests, including water absorption, gel strength and adhesion work were performed on the starch-based fluffy particles prepared in Examples 1 to 4, the carboxymethyl corn starch, the carboxymethyl potato starch and three comparative products (which were namely comparative products 1 to 3). Test results are shown in Table 1. The comparative product 1 is hemostatic powder prepared from potato starch by a cross-linking technology; the comparative product 2 is a product prepared according to the Chinese patent CN200610045441.9; and the comparative product 3 is a product prepared according to the Chinese patent CN200610053680.9.

$$\text{Water absorption rate} = \text{water absorption capacity (g or mL)/ powder weight (g).}$$

Water absorption speed: time required for adsorbing 10 g of water with 0.5 g of powder (second).

Gel strength: gel strength measured after the product is saturated with water and sealed standing for 48 hours; and

Adhesion work ($J/m^2$): the adhesion work of the gel is measured by a texture analyzer (manufacturer: Brookfield; model: CT3-100) after the product is saturated with water to a gel state.

Table 1 Property test results of different types of starch particles

| Sample | Example 1 | Example 2 | Example 3 | Example 4 | Carboxymethyl corn starch | Carboxymethyl potato starch | Comparative product 1 | Comparative product 2 | Comparative product 3 |
|---|---|---|---|---|---|---|---|---|---|
| Water absorption rate | 2000% | 2500% | 2200% | 2800% | 490% | 900% | 2400% | 150% | 250% |
| Water absorption speed | 22 | 19 | 24 | 18 | 120 | 60 | 20 | 300 | 120 |
| Gel strength (48 h, kPa) | 6.9 | 7.5 | 9 | 7.3 | 2.2 | 3.1 | Reliquefaction | 5.8 | Reliquefaction |
| Adhesion work (saturated absorption) | 72 | 78 | 90 | 74 | 22 | 28 | 70 | 120 | 20 |

It can be seen from Table 1 that, compared with the carboxymethyl corn starch without any treatment and the carboxymethyl potato starch without any treatment, the starch-based fluffy particles prepared in Examples 1 to 4 of the present disclosure all have significantly better effects in water absorption, gel strength and adhesion work. In addition, compared with the gel strength of carboxymethyl corn starch, carboxymethyl potato starch and Comparative products 1-3, the starch-based fluffy particles of Example 3 have more excellent gel strength due to containing polyoxyethylene, which indicates that the starch-based fluffy particles of Example 3 have superior elasticity, compared to the elasticity of carboxymethyl corn starch, carboxymethyl potato starch and Comparative products 1-3.

It can be seen from the water absorption rate and water absorption speed of Examples 2 to 4 and Comparative product 3 that, under the carboxymethyl potato starch as the main raw material, compared with Comparative product 3, the water absorption speed and water absorption rate of the starch-based fluffy particles of the present application are significantly improved.

Although the property of the German product is superior to those of the Chinese products 1 and 2, the property of the German product is inferior to those of the starch-based fluffy particles prepared in Examples 1 to 4, which indicates that the product prepared by the present disclosure has a better hemostatic property than conventional Chinese and foreign market products, and can be widely used in vivo and in vitro as a medical hemostatic material to achieve the purpose of fast and efficient hemostasis.

## Claims

1. A preparation method of a starch-based fluffy particle, comprising the following steps of:

   (1) adding water into starch or modified starch for gelatinization to obtain a starch paste;
   (2) adding the starch paste into an organic solvent that does not dissolve the starch paste so that the starch paste is dispersed into several precipitates in the organic solvent and the precipitates are swelled, stirring, standing and centrifuging to collect precipitates; and
   (3) freeze-drying, crushing and sieving the precipitates in sequence to prepare the starch-based fluffy particle.

2. The preparation method according to claim 1, wherein in step (1), the modified starch is at least one selected from the group consisting of etherified starch, carboxymethylated starch, esterified starch and crosslinked starch.

3. The preparation method according to claim 1, wherein in step (1), a mass ratio of the starch or the modified starch to the water in the starch paste is from 1: 3 to 1: 40.

4. The preparation method according to claim 1, wherein in step (2), the organic solvent is at least one selected from the group consisting of methanol, ethanol, propanol, butanol, acetone, isopropanol, and dimethyl sulfoxide.

5. The preparation method according to claim 4, wherein in step (2), the organic solvent is isopropanol, methanol, ethanol, a combination of isopropanol and methanol, dimethyl sulfoxide or acetone.

6. The preparation method according to claim 1, wherein the starch-based fluffy particle further comprises a hydrophilic polymer material selected in the group of sodium carboxymethyl cellulose, hyaluronic acid, polyoxyethylene, collagen.

7. The preparation method according to claim 1, wherein in step (2), the stirring is performed at a rotating speed of 100 r/min to 3,000 r/min, and lasts for 0.5 hour to 2 hours.

8. The preparation method according to claim 1, wherein in step (2), the centrifuging is performed at a rotating speed of 2,000 r/min to 5,000 r/min.

9. The preparation method according to claim 1, wherein in step (3), the freeze-drying comprises the steps of: freezing the precipitates at -30°C to -10°C for 3 hours to 24 hours first, and then freeze-drying the precipitates in a freeze dryer at -50°C to -30°C for 15 hours to 36 hours.

10. A starch-based fluffy particle prepared by the preparation method according to any one of claims 1 to 9, wherein a particle size of the starch-based fluffy particle is from 30 $\mu$m to 200 $\mu$m as measured by passing through a sieve of greater than or equal to 80 mesh and being retained by a sieve of less than or equal to 350 mesh.

**11.** A medical hemostatic material, comprising the starch-based fluffy particle according to claim 10.

**Patentansprüche**

**1.** Verfahren zur Herstellung eines flauschigen Partikels auf Stärkebasis, umfassend die folgenden Schritte:

(1) Zugabe von Wasser zu Stärke oder modifizierter Stärke zur Gelatinierung, um eine Stärkepaste zu erhalten;
(2) die Stärkepaste in ein organisches Lösungsmittel geben, das die Stärkepaste nicht auflöst, so dass die Stärkepaste in mehreren Niederschlägen im organischen Lösungsmittel dispergiert wird und die Niederschläge gequollen, gerührt, ruhen gelassen und zentrifugiert werden, um die Niederschläge zu sammeln; und
(3) Gefriertrocknen, Mahlen und Sieben der Niederschläge nacheinander, um die flauschigen Partikel auf Stärkebasis herzustellen.

**2.** Herstellungsverfahren nach Anspruch 1, wobei in Schritt (1) ist die modifizierte Stärke mindestens eine Stärke, ausgewählt aus der Gruppe bestehend aus veretherter Stärke, carboxymethylierter Stärke, veresterter Stärke und vernetzter Stärke.

**3.** Herstellungsverfahren nach Anspruch 1, wobei in Schritt (1) ein Massenverhältnis von Stärke oder modifizierter Stärke zu Wasser in der Stärkepaste zwischen 1:3 und 1:40 beträgt.

**4.** Herstellungsverfahren nach Anspruch 1, wobei in Schritt (2) ist das organische Lösungsmittel mindestens ein Lösungsmittel, ausgewählt aus der Gruppe bestehend aus Methanol, Ethanol, Propanol, Butanol, Aceton, Isopropanol und Dimethylsulfoxid.

**5.** Herstellungsverfahren nach Anspruch 4, wobei in Schritt (2) ist das organische Lösungsmittel Isopropanol, Methanol, Ethanol, eine Kombination aus Isopropanol und Methanol und Dimethylsulfoxid oder Aceton.

**6.** Herstellungsverfahren nach Anspruch 1, wobei das flauschige Teilchen auf Stärkebasis außerdem ein hydrophiles Polymermaterial umfasst, ausgewählt aus der Gruppe bestehend aus Natriumcarboxymethylcellulose, Jaluronsäure, Polyoxyethylen und Kollagen.

**7.** Herstellungsverfahren nach Anspruch 1, wobei in Schritt (2) wird das Rühren bei einer Rotationsgeschwindigkeit von 100 U/min bis 3.000 U/min durchgeführt und 0,5 Stunden bis 2 Stunden dauert.

**8.** Herstellungsverfahren nach Anspruch 1, wobei in Schritt (2) die Zentrifugation bei einer Rotationsgeschwindigkeit von 2.000 U/min bis 5.000 U/min durchgeführt wird.

**9.** Herstellungsverfahren nach Anspruch 1, wobei in Schritt (3) die Lyophilisierung die folgenden Schritte umfasst: Einfrieren der Niederschläge zwischen -30°C und -10°C für zunächst 3 bis 24 Stunden und dann Gefriertrocknung der Niederschläge in einem Lyophilisator bei -50°C bis -30°C für 15 bis 36 Stunden.

**10.** Flauschige Partikel auf Stärkebasis, hergestellt durch das Herstellungsverfahren nach einem der Ansprüche 1 bis 9, wobei die Größe der flauschigen Partikel auf Stärkebasis zwischen 30 $\mu$m und 200 $\mu$m liegt, gemessen durch Passieren durch einen Filter, der größer als oder gleich 80 Mesh ist und dessen Rückhaltung durch einen Filter kleiner oder gleich 350 Mesh erfolgt.

**11.** Medizinisches hämostatisches Material, umfassend das flauschige Teilchen auf Stärkebasis nach Anspruch 10.

**Revendications**

**1.** Procédé de préparation d'une particule duveteuse à base d'amidon, comprenant les étapes suivantes :

(1) ajouter de l'eau dans de l'amidon ou de l'amidon modifié pour la gélatinisation afin d'obtenir une pâte d'amidon;
(2) ajouter la pâte d'amidon dans un solvant organique qui ne dissout pas la pâte d'amidon de sorte que la pâte d'amidon soit dispersée en plusieurs précipités dans le solvant organique et que les précipités soient gonflés, agités, laissés au repos et centrifugés pour recueillir les précipités ; et

(3) lyophiliser, broyer et tamiser les précipités en séquence pour préparer la particule duveteuse à base d'amidon.

2. Procédé de préparation selon la revendication 1, dans lequel à l'étape (1), l'amidon modifié est au moins un amidon choisi dans le groupe constitué de l'amidon éthérifié, de l'amidon carboxyméthylé, de l'amidon estérifié et de l'amidon réticulé.

3. Procédé de préparation selon la revendication 1, dans lequel à l'étape (1), un rapport massique de l'amidon ou de l'amidon modifié à l'eau dans la pâte d'amidon est compris entre 1:3 et 1:40.

4. Procédé de préparation selon la revendication 1, dans lequel à l'étape (2), le solvant organique est au moins un solvant choisi dans le groupe constitué du méthanol, de l'éthanol, du propanol, du butanol, de l'acétone, de l'isopropanol et du diméthylsulfoxyde.

5. Procédé de préparation selon la revendication 4, dans lequel à l'étape (2), le solvant organique est de l'isopropanol, du méthanol, de l'éthanol, d'une combinaison d'isopropanol et de méthano, et de diméthylsulfoxyde ou de l'acétone.

6. Procédé de préparation selon la revendication 1, dans lequel la particule duveteuse à base d'amidon comprend en outre un matériau polymère hydrophile, choisi dans le groupe constitué de carboxyméthylcellulose de sodium, acide jaluronique, polyoxyéthylène, collagène.

7. Procédé de préparation selon la revendication 1, dans lequel à l'étape (2), l'agitation est réalisée à une vitesse de rotation de 100 tr/min à 3 000 tr/min et dure 0,5 heure à 2 heures.

8. Procédé de préparation selon la revendication 1, dans lequel à l'étape (2), la centrifugation est réalisée à une vitesse de rotation de 2.000 tr/min à 5.000 tr/min.

9. Procédé de préparation selon la revendication 1, dans lequel à l'étape (3), la lyophilisation comprend les étapes consistant à: congeler les précipités entre -30°C et -10°C pendant 3 heures à 24 heures dans un premier temps, et puis à lyophiliser les précipités au lyophilisateur entre -50°C et -30°C pendant 15 heures à 36 heures.

10. Particule duveteuse à base d'amidon préparée par le procédé de préparation selon l'une quelconque des revendications 1 à 9, dans laquelle la taille des particules duveteuses à base d'amidon est comprise entre 30 $\mu$m et 200 um, mesurée après passage à travers un filtre plus grand ou égal à 80 mailles et étant retenue par un filtre plus petit ou égal à 350 mailles.

11. Matériau hémostatique médical, comprenant la particule duveteuse à base d'amidon selon la revendication 10.

FIG. 1

FIG. 2

FIG. 3

FIG. 4

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 200710141944, Xin Ji
- CN 101423620 A
- CN 200610053680
- CN 200610045441